# EUROPEAN PATENT APPLICATION

(11) **EP 2 153 774 A1**
(43) Date of publication of application: **17.02.2010**
(21) Application number: 08752231.4
(22) Date of filing: 29.04.2008
(51) Int. Cl.: A61B 5/151, A61B 5/1491, A61B 5/157, G01N 27/327

(54) **ANALYZING SYSTEM**

(30) Priority: 29.04.2007 JP 2007120395
(71) Applicant: Arkray, Inc., Kyoto-shi, Kyoto 601-8045 (JP)
(72) Inventor: KUSAKA, Yasuhide, Kyoto-shi Kyoto 601-8045 (JP); KADO, Kotaro, Kyoto-shi Kyoto 601-8045 (JP); WAKITA, Kazuhiro, Kyoto-shi Kyoto 601-8045 (JP); SATO, Yoshiharu, Kyoto-shi Kyoto 601-8045 (JP)
(74) Representative: HOFFMANN EITLE
(86) International application number: PCT/JP2008/058221
(87) International publication number: WO 2008/136472

(57) **Abstract**

This invention is regard to an analysis system comprising a laser beam oscillator 60 which emits laser beam for issuing bodily fluid from a skin, and one or more analysis tools 2 which are used for analyzing specific ingredient in the bodily fluid and have a through hole 23 through which the laser beam passes. The analysis tool 2 includes a plurality of conductors 20, 21 laminated in a state where they are electrically insulated from each other. The analysis tool 2 further includes a insulation layer 26 interposed between adjacent conductors 20, 21, and a insulation layers 27,28 which covers an outer surface of at least one of two outermost conductors located on the outermost portions among the plurality of conductors 20,21.

## Description

### TECHNICAL FIELD

The present invention relates to an analysis system for issuing bodily fluid from a skin by laser beam, and for electrochemically analyzing specific ingredient (such as glucose, cholesterol and lactic acid) in the bodily fluid.

### BACKGROUND TECHNOLOGY

When measuring concentration of glucose or the like in blood, a method of utilizing a single-use analysis tool is employed as a simple method (see patent document 1, for example). As the analysis tool, there is one capable of carrying out analysis electrochemically.

A sample such as blood can be obtained by incising a skin using a lancet, for example. It is general to impale a skin with a puncture needle as the lancet, but there is also a lancet capable of issuing blood from a skin by irradiating the skin with laser beam (see patent document 2, for example).

According to a method using the analysis tool and the lancet, it is necessary for a user to apply blood issued from a skin by the lancet onto a spot of the analysis tool. This puts a heavy load on a user, or some users can not even apply blood on a spot of the analysis tool appropriately.

Patent Document 1: Japanese Patent Publication No. H8-10208
Patent Document 2: Japanese Patent Application Laid-open No. H4-314428

### DISCLOSURE OF THE INVENTION

### PROBLEMS TO BE SOLVED BY THE INVENTION

It is an object of the present invention to make it possible to appropriately supply bodily fluid such as blood to an analysis tool without putting a load on a user.

### MEANS FOR SOLVING THE PROBLEM

The present invention provides an analysis system comprising a laser beam oscillator which emits laser beam for issuing bodily fluid from a skin, one or more analysis tools which are used for analyzing specific ingredient in the bodily fluid and have a through hole through which the laser beam passes, wherein the analysis tool includes a plurality of conductors laminated in a state where they are electrically insulated from each other.

For example, the analysis tool further includes a first insulation layer interposed between adjacent conductors, and one or a pair of second insulation layers which covers an outer surface of at least one of two outermost conductors located on the outermost portions among the plurality of conductors. The second insulation layer hermetically closes an interior of the through hole. In this case, it is preferable that the second insulation layer is made of material formed with an opening by the laser beam. It is preferable that the second insulation layer includes one or more holes from which a surface of the outermost conductor is exposed.

The analysis tool has symmetric shapes with respect to a center line as viewed from above. In this case, it is preferable that the plurality of holes are formed at symmetric positions with respect to the center line.

The two outermost conductors have the same or almost the same thicknesses. In this case, it is preferable that a plurality of holes in the pair of second insulation layers are formed at the same or almost the same positions as viewed from above.

The analysis tool may have one or more positioning portions. The positioning portion may be a convex portion or a concave portion.

The through hole is formed at a center or substantially a center of the analysis tool as viewed from above. In this case, it is preferable that one of the holes is annularly formed such as to surround the through hole.

The analysis tool may be formed asymmetrically as viewed from above and for example, the analysis tool may be formed into a trapezoidal shape.

The analysis tool may further include a heating element for heating the plurality of conductors.

The analysis system of the invention further includes a connector having a plurality of terminals which are brought into contact with the plurality of conductors of the analysis tool.

In the analysis system of the invention, the plurality of analysis tools are disposed in a state where they are laminated on one another. In this case, it is preferable that the analysis system further includes an analysis tool supply mechanism for supplying analysis tools to the connector one by one.

The analysis tool supply mechanism electromagnetically supplies the analysis tool to the connector. In this case, at least one of the plurality of conductors is made of magnetic material. The analysis tool supply mechanism includes first and second electromagnets, at least a portion of the analysis tool is magnetized by the first electromagnet and then, a polarity of the first electromagnet is reversed, repulsion is generated to the analysis tool, a polarity of the second electromagnet is set same with that of the first electromagnet, and an attraction force is generated to the analysis tool.

The plurality of analysis tools may be connected to one another in a form of an array. In this case, it is preferable that the plurality of analysis tools includes cutting slits formed between the adjacent analysis tools so that the analysis tool can be cut.

### BRIEF DESCRIPTION OF THE DRAWINGS

Fig. 1 is an overall perspective view showing one example of an entire analysis apparatus in an analysis system according to the present invention;
Fig. 2 is a sectional view taken along the line II-II in Fig. 1;
Fig. 3 is an overall perspective view showing one example of a biosensor in the analysis apparatus shown in Fig. 1;
Fig. 4 is a sectional view taken along the line IV-IV in Fig. 3;
Fig. 5 is a sectional view showing an essential portion for explaining a connector and a lasing mechanism in the analysis apparatus shown in Fig. 1;
Figs. 6A to 6C are sectional views showing an essential portion for explaining a sensor supply mechanism in the analysis apparatus shown in Fig. 1;
Figs. 7A and 7B are sectional views showing an essential portion for explaining a sensor detection mechanism in the analysis apparatus shown in Fig. 1;
Figs. 8A and 8B are sectional views showing an essential portion for explaining another example of the sensor detection mechanism;
Fig. 9 is an overall perspective view for explaining another example of the biosensor;
Fig. 10 is a partial exploded perspective view of the biosensor shown in Fig. 9;
Fig. 11 is a sectional view taken along the line XI-XI in Fig. 9;
Fig. 12 is a sectional view taken along the line XII-XII in Fig. 9;
Fig. 13 is a sectional view corresponding to Fig. 5 for explaining effect of the biosensor shown in Fig. 9;
Fig. 14 is a sectional view for explaining another example of the biosensor;
Fig. 15 is an overall perspective view for explaining another example of the biosensor;
Fig. 16 is a sectional view taken along the line XVI-XVI in Fig. 15;
Fig. 17 is an overall perspective view for explaining another example of the biosensor;
Fig. 18 is a sectional view taken along the line XVIII-XVIII in Fig. 17;
Fig. 19 is an overall perspective view for explaining another example of the biosensor;
Fig. 20 is a sectional view taken along the line XX-XX in Fig. 19;
Fig. 21 is an overall perspective view for explaining another example of the biosensor;
Fig. 22 is an overall perspective view for explaining another example of the biosensor;
Fig. 23 is an overall perspective view for explaining another example of the biosensor;
Fig. 24 is an overall perspective view for explaining another example of the biosensor;
Fig. 25 is an overall perspective view for explaining another example of the biosensor;
Fig. 26 is a sectional view taken along the line XXVI-XXVI in Fig. 25; and
Fig. 27 is a sectional view taken along the line XXVII-XXVII in Fig. 25.

### DESCRIPTION OF REFERENCE SYMBOLS

- 1: analysis apparatus
- 2: biosensor (analysis tool)
- 20: working electrode (outermost conductor)
- 21: counter electrode (outermost conductor)
- 23: capillary (through hole of analysis tool)
- 23B: opening (of capillary)
- 25: heating layer (heating element)
- 26: insulation layer (first insulation layer)
- 27: insulation layer (second insulation layer)
- 27B, 28B, 27B', 28B', 27B", 28B": hole (of insulation layer)
- 4, 4', 4": connector
- 41: movable body (moving portion) (of connector)
- 41': block (moving portion) (of connector)
- 41B: through hole (of movable body)
- 42, 43: measuring terminal (terminal)
- 50: first electromagnet (of sensor supply mechanism)
- 51: second electromagnet (of sensor supply mechanism)
- 60: laser beam oscillator
- 7, 7', 7": sensor detection mechanism (detection mechanism)
- 70': detection terminal
- 71: switch (of sensor detection mechanism)
- 8A, 8B, 8C, 8D, 8E, 8F, 8G, 8H, 8I: biosensor
- 80A: cover
- 801: (cutting) slit
- 80E, 80F: positioning convex portion (positioning portion)
- 81A: discharge passage
- 82A: slit (of insulation layer)

### PREFERRED EMBODIMENT OF THE INVENTION

An analysis system according to the present invention will be described with reference to the drawings.

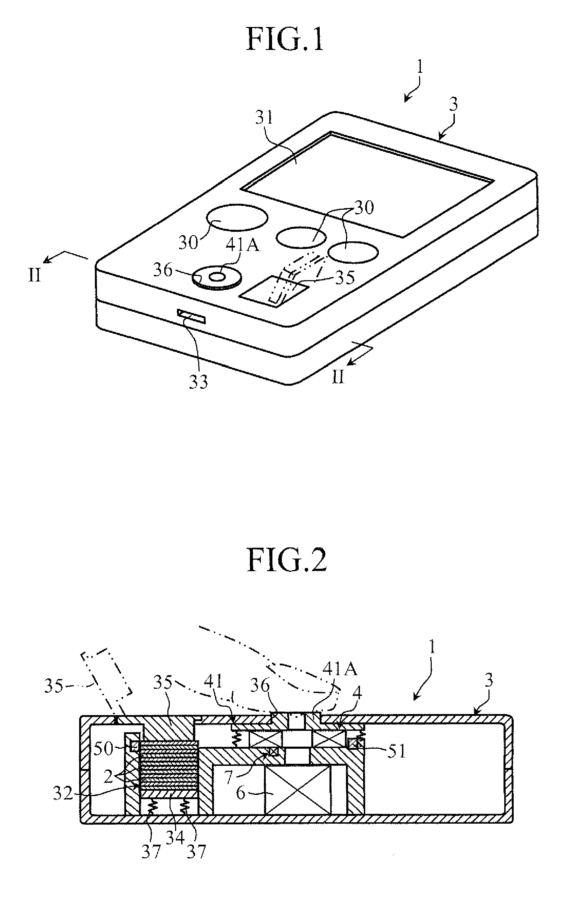

An analysis apparatus 1 shown in Figs. 1 and 2 is for analyzing a sample by an electrochemical method using biosensors 2. The analysis apparatus 1 is constituted as a portable type apparatus that can easily be carried. The analysis apparatus 1 accommodates therein a plurality of biosensors 2, and includes a casing 3, a connector 4, a sensor supply mechanisms 50 and 51, a lasing mechanism 6 and a sensor detection mechanism 7.

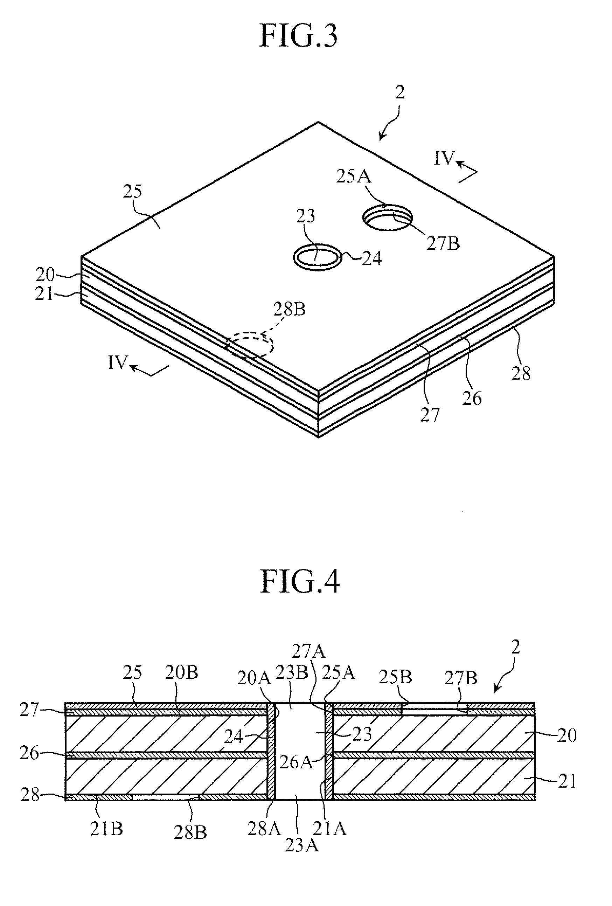

As shown in Figs. 3 and 4, the biosensors 2 are constituted as single-use biosensors. The biosensors 2 are used for analyzing specific ingredient (such as glucose, cholesterol and lactic acid) in bodily fluid such as blood and interstitial fluid. The biosensor 2 is formed into a rectangular plate-like shape as a whole, and has a size of (2 to 10 mm) × (2 to 10 mm) × (0.5 to 2 mm), for example. The biosensor 2 includes a working electrode 20 and a counter electrode 21 which are laminated on each other, and further includes a capillary 23, a reagent layer 24 and a heating layer 25.

The working electrode 20 and the counter electrode 21 apply voltage to bodily fluid introduced into the capillary 23, and are utilized to measure response current at that time. The working electrode 20 and the counter electrode 21 include through holes 20A and 21A and are formed into the same or almost the same shape. The through holes 20A and 21A define the capillary 23, and are formed into a circle having a diameter of 0.2 to 1 mm at central portions of the working electrode 20 and the counter electrode 21. The working electrode 20 and the counter electrode 21 are made of conductive magnetic material such as nickel, and formed into a size of (2 to 10 mm) × (2 to 10 mm) × (0.2 to 1 mm).

An insulation layer 26 is interposed between the working electrode 20 and the counter electrode 21, and the working electrode 20 and the counter electrode 21 are bonded to each other through the insulation layer 26. A through hole 26A defining the capillary 23 is formed in a central portion of the insulation layer 26, and a thickness of the insulation layer 26 is formed into 20 to 100 µm by a known hot-melt sheet. A diameter of the through hole 26A is the same or almost the same as those of the through holes 20A and 21A of the working electrode 20 and the counter electrode 21.

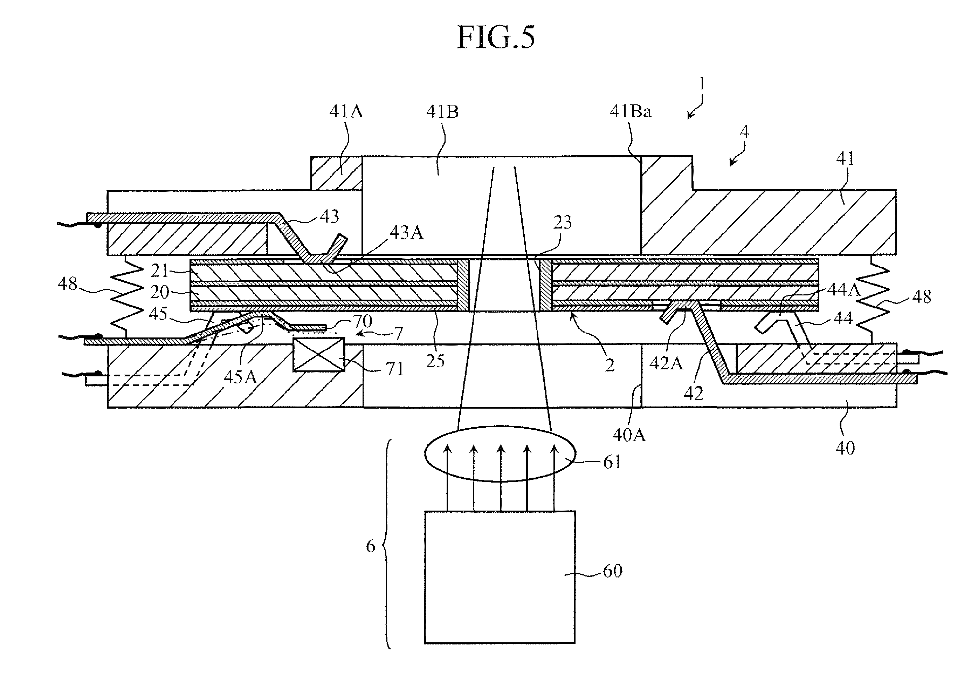

Insulation layers 27 and 28 are formed on surfaces 20B and 21B of the working electrode 20 and the counter electrode 21. These insulation layers 27 and 28 are for retraining bodily fluid from adhering to the surfaces 20B and 21B of the working electrode 20 and the counter electrode 21. The insulation layers 27 and 28 also have through holes 27A and 28A like the insulation layer 26 by a known hot-melt sheet. Diameters of the through holes 27A and 28A are the same or almost the same as those of the through holes 20A and 21A of the working electrode 20 and the counter electrode 21. The insulation layers 27 and 28 are formed with holes 27B and 28B from which the surface 20B or 21B of the working electrode 20 or the counter electrode 21 is exposed. Later-described measuring terminals 42 and 43 (see Fig. 5) of the connector 4 can come into contact with the working electrode 20 or the counter electrode 21 through the holes 27B and 28B.

The capillary 23 is for moving bodily fluid introduced from an opening 23A toward an opening 23B utilizing capillary action and for holding the bodily fluid therein. The capillary 23 permits laser beam from entering from the later-described lasing mechanism 6 (see Fig. 5). The capillary 23 is defined by the through holes 20A, 21A and 26A to 28A of the working electrode 20, the counter electrode 21 and the insulation layers 26 to 28. The volume is set to 0.08 to 10 µL, for example.

The reagent layer 24 includes a reagent required for analysis of specific ingredient in bodily fluid, and covers an inner surface of the capillary 23. The reagent layer 24 includes electron transport material and oxyreductase, and is formed into a solid object which easily melts in bodily fluid. When bodily fluid is introduced into the capillary 23, the reagent layer 24 melts, and a liquid-phase reaction system including electron transport material, oxyreductase and bodily fluid is constituted in the capillary 23.

Material as the oxyreductase is selected depending upon kinds of specific ingredient to be analyzed. For example, when glucose is to be analyzed, glucose dehydrogenase (GDH) or glucose oxidase (GOD) can be used. Material as the electron transport material, ruthenium complex or iron complex can be used. Typically, [Ru(NH₃)₆]Cl₃ or K₃[Fe(CN)₆] can be used.

The heating layer 25 is for adjusting a temperature of the liquid-phase reaction system in the capillary 23, and covers substantially the entire insulation layer 27. The heating layer 25 has a through hole 25A and a hole 25B. The through hole 25A is brought into communication with the through hole 27A of the insulation layer 27, and the hole 25B is in communication with the hole 27B of the insulation layer 27. The entire heating layer 25 is made of resistance material. As the resistance material, various known materials such as iron-chromium-aluminum-based material and nickel-chromium-based material can be used.

It is not always necessary that the heating layer 25 covers substantially the entire surface 20A of the working electrode 20, and the heating layer 25 may be provided by pattern-forming a bellows wiring.

The casing 3 shown in Figs. 1 and 2 defines an outward appearance of the analysis apparatus 1, and includes a plurality of operation buttons 30, a display panel 31, a sensor accommodating portion 32 and a waste vent 33. The plurality of operation buttons 30 produce signals for carrying out the analysis operation, and for carrying out various setting operations (such as setting of analysis condition and input of ID of a subject). An analysis result, an error, operating procedure and an operating status at the time of setting operation are displayed on the display panel 31. The plurality of biosensors 2 are laminated and accommodated in the sensor accommodating portion 32. The sensor accommodating portion 32 includes a mounting portion 34 and a lid 35 which can open and close. The mounting portion 34 is biased upward by a coil spring 37 (toward the lid 35). A biosensor 2 that was used for analysis is discarded from the analysis apparatus 1 through the waste vent 33.

As shown in Fig. 5, the connector 4 holds a biosensor 2 to be analyzed, and applies voltage to a location between the working electrode 20 and the counter electrode 21 of the biosensor 2 or to the heating layer 25. The connector 4 includes a fixed body 40, a movable body 41, the measuring terminals 42 and 43 and heating terminals 44 and 45.

The fixed body 40 supports the measuring terminal 42 and the heating terminals 44 and 45, and includes a through hole 40A. The through hole 40A permits laser beam to enter from the lasing mechanism 6, and the later-described sensor detection mechanism 7 (elastic body 70 and switch 71) is disposed in the fixed body 40.

The movable body 41 supports the measuring terminal 43. The movable body 41 is connected to the fixed body 40 through a coil spring 48, the movable body 41 is biased upward and the movable body 41 can vertically move. The movable body 41 includes a convex portion 41A and a through hole 41B. A skin such as a fingertip is pushed against the convex portion 41A when extracting bodily fluid, and the convex portion 41A is exposed from a through hole 36 (see Fig. 1) of the casing 3. That is, if a skin such as a fingertip is pushed against the convex portion 41A, the movable body 41 is moved downward. The through hole 41B permits laser beam to enter from the lasing mechanism 6, and the through hole 41B continuously extends to the convex portion 41A, and is in communication with outside of the apparatus at an end surface of the convex portion 41A. That is, the opening 41Ba functions as a bodily fluid extracting opening of the through hole 41B.

The terminals 42 to 45 are constituted as leaf springs. The measuring terminals 42 and 43 are for applying voltage between the working electrode 20 and the counter electrode 21 of the biosensor 2. The measuring terminal 42 comes into contact with the working electrode 20, a contact 42A projects upward. The measuring terminal 43 comes into contact with the counter electrode 21, and a contact 43A projects downward. The heating terminals 44 and 45 apply voltage to the heating layer 25 of the biosensor 2 to heat the heating layer 25. The contacts 44A and 45A of the heating terminals 44 and 45 project upward, and come into contact with the heating layer 25.

In the connector 4, the contacts 42A, 44A and 45A of the measuring terminal 42 constituted as a leaf spring and the heating terminals 44 and 45 project upward from the fixed body 40, and the contact 43A of the measuring terminal 43 projects downward from the movable body 41. Therefore, in the connector 4, the biosensor 2 can be held between the fixed body 40 and the movable body 41.

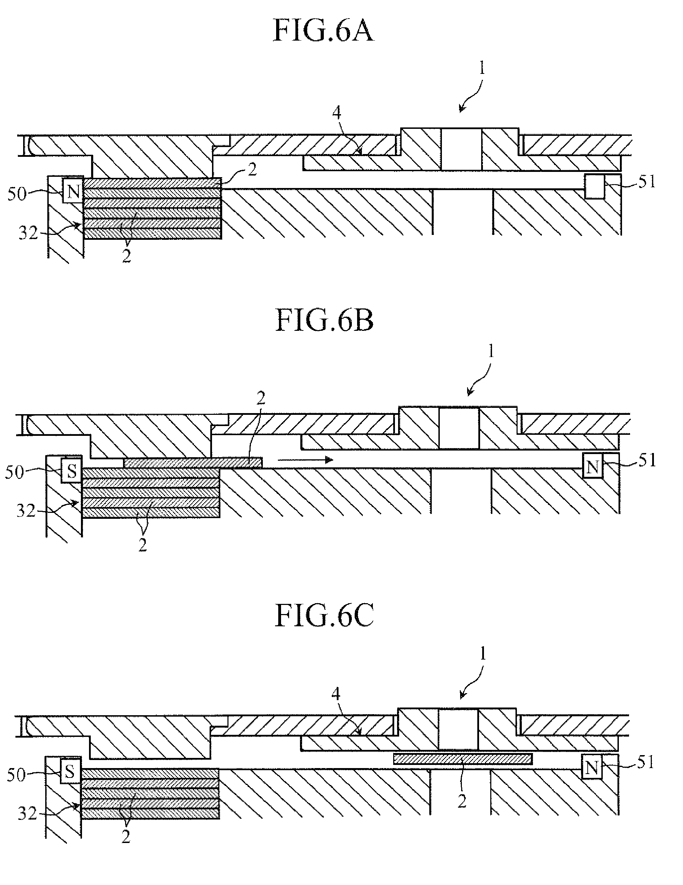

As shown in Figs. 6A to 6C, the sensor supply mechanisms 50 and 51 supply, to the connector 4, the uppermost one of the plurality of biosensors 2 laminated on the sensor accommodating portion 32. The sensor supply mechanisms 50 and 51 include electromagnets 50 and 51, respectively. The electromagnet 50 is provided adjacent to the sensor accommodating portion 32, and the electromagnet 51 is provided adjacent to the connector 4. The electromagnet 50 magnetizes the biosensor 2, and applies repulsion between the magnetized biosensor 2 and the electromagnet 50. The electromagnet 51 applies an attraction force between the magnetized biosensor 2 and the electromagnet 51.

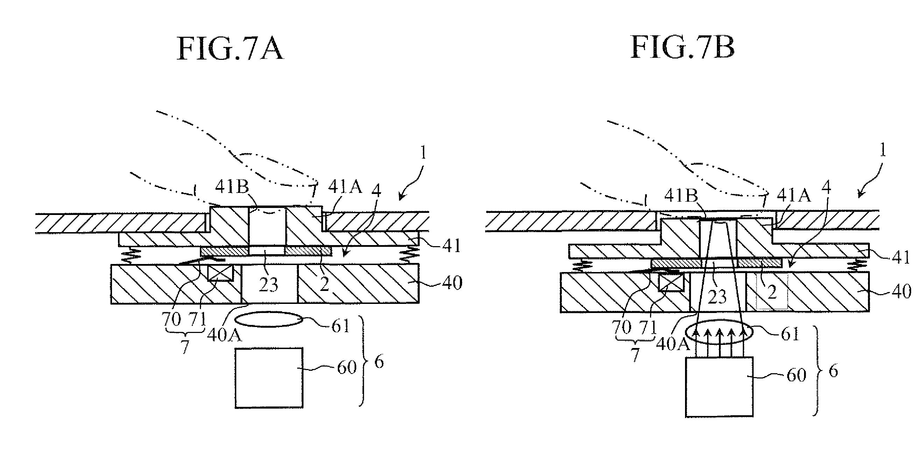

As shown in Figs. 7A and 7B, when issuing bodily fluid such as blood from a skin, the lasing mechanism 6 emits laser beam to be emitted to the skin. The lasing mechanism 6 includes a laser beam oscillator 60 such as a laser diode and a condensing lens 61.

As shown in Figs. 5, 7A and 7B, the sensor detection mechanism 7 is for detecting whether the biosensor 2 exists in a target position of the connector 4, and includes the elastic body 70 and the switch 71. The elastic body 70 is fixed to the fixed body 40 in the connector 4, and is short-circuited with the switch 71. The elastic body 70 turns the switch 71 ON when the movable body 41 (biosensor 2) moves downward. The switch 71 is for turning predetermined motion of the analysis apparatus 1 ON and OFF. When the switch 71 is ON, the switch 71 controls the laser beam oscillator 60 and emits laser beam.

The elastic body 70 may be fixed to the movable body 41. The elastic body 70 may have elasticity due to a shape other than a leaf spring or properties of material.

Next, operation of the analysis apparatus 1 will be described.

As shown in Figs. 6A to 6C, in the analysis apparatus 1, when a plurality of biosensors 2 are set in the sensor accommodating portion 32 or analysis is completed, the biosensors 2 are supplied to the connector 4 from the sensor accommodating portion 32 by the sensor supply mechanisms 50 and 51.

More concretely, in the sensor supply mechanisms 50 and 51, as shown in Fig. 6A, the biosensor 2 is magnetized by the electromagnet 50. In the illustrated example, in the electromagnet 50, the north pole is adjacent to the biosensor 2, a side of the biosensor 2 close to the electromagnet 50 is magnetized with south pole and a side of the biosensor 2 farther to the electromagnet 50 is magnetized with the north pole. At that time, no magnetic pole is generated in the electromagnet 51.

Next, as shown in Figs. 6B and 6C, the polarity of the electromagnet 50 is reversed and repulsion is generated between the biosensor 2 and the electromagnet 50. The polarity is generated in the electromagnet 51, and an attraction force is generated between the biosensor 2 and the electromagnet 50 as reversed polarity. The biosensor 2 is moved toward the connector 4 by the repulsion of the electromagnet 50 and the attraction force by the electromagnet 51.

As shown in Fig. 5, in the connector 4, terminals 42 to 45 constituted as the leaf springs project from the fixed body 40 and the movable body 41 and thus, a load for sandwiching the biosensor 2 is applied to the connector 4. On the other hand, as shown in Figs. 3 and 4, in the biosensor 2, the holes 27B and 28B are formed in the insulation layers 27 and 28 through which the working electrode 20 and the counter electrode 21 are exposed. Therefore, the movement of the biosensor 2 is stopped by the step between the holes 27A and 28B. At that time, the measuring terminal 42 of the connector 4 comes into contact with the working electrode 20, the measuring terminal 43 comes into contact with the counter electrode 21, and the heating terminals 44 and 45 come into contact with the heating layer 25.

A detection mechanism for detecting that the biosensor 2 is mounted on the connector 4 is provided, and when the biosensor 2 may be detected by the detection mechanism, polarities may not be generated in the electromagnets 50 and 51, and movement of the biosensor 2 may be stopped. The detection mechanism in this case may employ such a structure that voltage is applied between the heating terminals 44 and 45 in the connector 4, and current-carrying states of the heating terminals 44 and 45 are checked or confirmed.

The sensor supply mechanisms 50 and 51 are not limited to those having the electromagnets 50 and 51, and may utilize a known actuator for example. In this case, in the biosensor 2, it is not always necessary that the working electrode 20 and the counter electrode 21 are made of magnetic material.

As shown in Figs. 7A and 7B, when analysis of specific ingredient in bodily fluid is to be carried out using the analysis apparatus 1, a skin such as a fingertip is pushed against the convex portion 41A of the movable body 41, and the movable body 41 is moved downward. With this, the elastic body 70 in the sensor detection mechanism 7 is moved downward together with the movable body 41 (biosensor 2). If the elastic body 70 moves downward, the elastic body 70 turns the switch 71 ON, and the power supply of the analysis apparatus 1 is turned ON. At that time, laser beam is emitted from the lasing mechanism 6.

The biosensor 2 includes the capillary 23. The fixed body 40 and the movable body 41 include through holes 40A and 41B. Thus, a skin placed on the convex portion 41A is irradiated with laser beam emitted from the laser beam oscillator 60. When a skin is irradiated with laser beam, bodily fluid such as blood is issued from the skin. At that time, since the skin is pushed against the convex portion 41A, the skin is congested, and issuing phenomenon of bodily fluid such as blood is accelerated.

Bodily fluid from a skin is introduced into the capillary 23 by capillary action generated in the capillary 23 of the biosensor 2. The reagent layer 24 is melted in the capillary 23, and the liquid-phase reaction system is constituted.

When the switch 71 is turned ON, as shown in Fig. 5, voltage is applied between the measuring terminals 42 and 43 in the connector 4 and between the heating terminals 44 and 45. If voltage is applied between the heating terminals 44 and 45, the heating layer 25 is heated. Heat of the heating layer 25 is transmitted to the working electrode 20 and the counter electrode 21, and bodily fluid introduced into the capillary 23 is heated. With this, the bodily fluid in the capillary 23 is heated to a target temperature.

If voltage is applied between the measuring terminals 42 and 43 and between the heating terminals 44 and 45, voltage is applied between the working electrode 20 and the counter electrode 21, and voltage is also applied to the liquid-phase reaction system. With this, specific ingredient such as glucose in the bodily fluid is reduced (electrons are taken out) by oxyreductase, and the electrons are supplied to the working electrode 20 through the electron transport material. An amount of electrons supplied to the working electrode 20 is measured as response current through the measuring terminals 42 and 43. In the analysis apparatus 1, concentration of specific ingredient such as glucose is calculated based on the response current. A result of the calculation is displayed on the display panel 31 shown in Fig. 1.

When the analysis of bodily fluid is completed, used biosensor 2 is discarded through the waste vent 33. Such biosensor 2 may be discarded automatically by a discarding mechanism provided in the analysis apparatus 1 or a user may discard the biosensor 2 manually by operating a lever. When a used biosensor 2 is discarded, a new biosensor 2 is supplied to the connector 4 by the sensor supply mechanisms 50 and 51.

In the analysis apparatus 1, bodily fluid issued from a skin by the laser beam is supplied to the biosensor 2 in its intact extracted state. Therefore, it is unnecessary to separate apparatuses for extracting bodily fluid and for analyzing the bodily fluid, blood can easily be sucked by the biosensor 2, and a load on a user is reduced.

In the analysis apparatus 1, the switch 71 is turned ON when a skin is pushed against the convex portion 41A of the movable body 41 in the connector 4. Therefore, laser beam is emitted only during movement of the convex portion 41A by a skin, it is possible to suppress unintentional emission of laser beam. In the case of a structure in which the switch 71 is turned ON by pushing the skin, only necessary circuit is operated only while the skin is pushed and thus, power consumption can be suppressed and running cost can be reduced.

It is not always necessary to use a biosensor 2 that is previously accommodated in the analysis apparatus 1, and the biosensor 2 can be mounted on the connector 4 in the analysis apparatus 1 at the time of analysis.

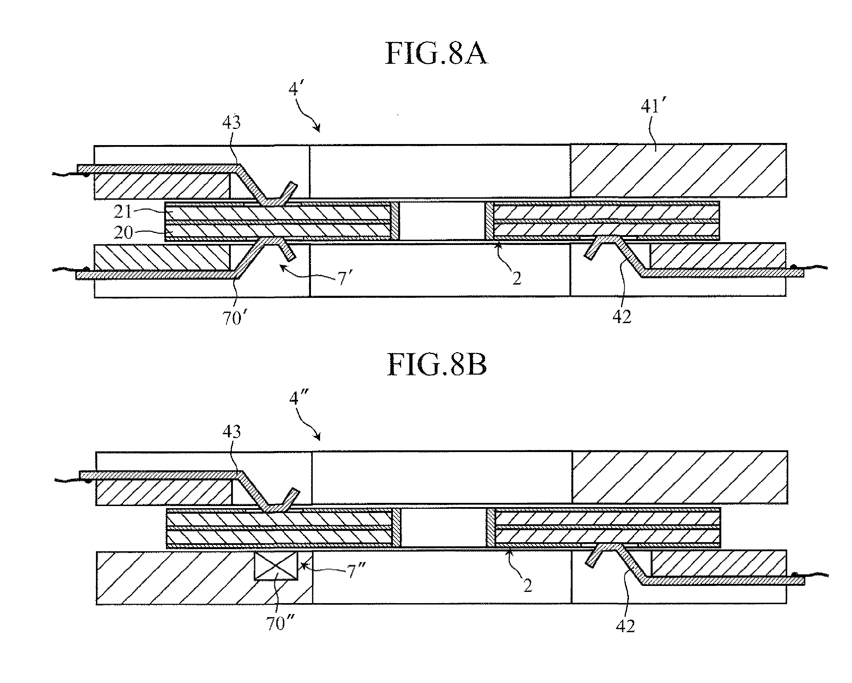

Next, another example of the sensor detection mechanism will be described with reference to Figs. 8A and 8B. In Figs. 8A and 8B, the same elements as the analysis apparatus 1 and the biosensors 2 explained previously with reference to Figs. 1 to 7 are designated with the same symbols, and redundant explanation will be omitted below.

A sensor detection mechanism 7' shown in Fig. 8A includes a detection terminal 70' in addition to the measuring terminals 42 and 43 in a connector 4'. The detection terminal 70' comes into contact with the working electrode 20 of the biosensor 2. That is, the detection terminal 70' detects whether the detection terminal 70' and the measuring terminal 42 are short circuited through the working electrode 20, thereby making it possible to detect that a biosensor 2 is supplied to the connector 4'. The measuring terminal 42 in this case also functions as a detection terminal.

The sensor detection mechanism 7' may employ such a structure that an upper block 41' in the connector 4' can move, and the block 41' is made to move downward, thereby bringing the detection terminal 70' into contact with the working electrode 20, or the sensor detection mechanism 7' may employ such a structure that a biosensor 2 is mounted on the connector 4', thereby bringing the detection terminal 70' into contact with the working electrode 20.

Short circuit between the detection terminal and the measuring terminal 43 may be detected utilizing the counter electrode 21, or short circuit between a pair of detection terminals used only for detection in addition to the measuring terminals 42 and 43 may be detected utilizing the working electrode 20 or the counter electrode 21.

A sensor detection mechanism 7" shown in Fig. 8B detects that a biosensor 2 is supplied to a connector 4" by an optical technique. The connector 4" is provided with an optical sensor 70" such as a photosensor. The optical sensor 70" recognizes a predetermined locations in a biosensor 2, thereby detecting that a biosensor 2 is supplied to the connector 4".

In the sensor detection mechanisms 7' and 7" shown in Figs. 8A and 8B, it is possible to employ a structure capable of emitting laser beam from the lasing mechanism 6 (see Fig. 7) when it is detected that a biosensor 2 is supplied to the connector 4' or 4". That is, it is possible to suppress erroneous emission of laser beam so that laser beam is not emitted from the lasing mechanism 6 unless a biosensor 2 is mounted on the connector 4' or 4".

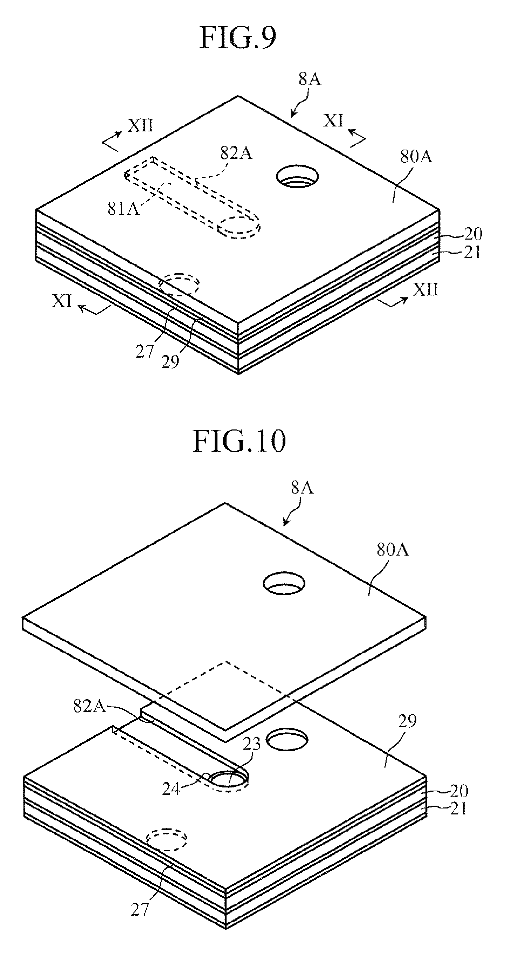

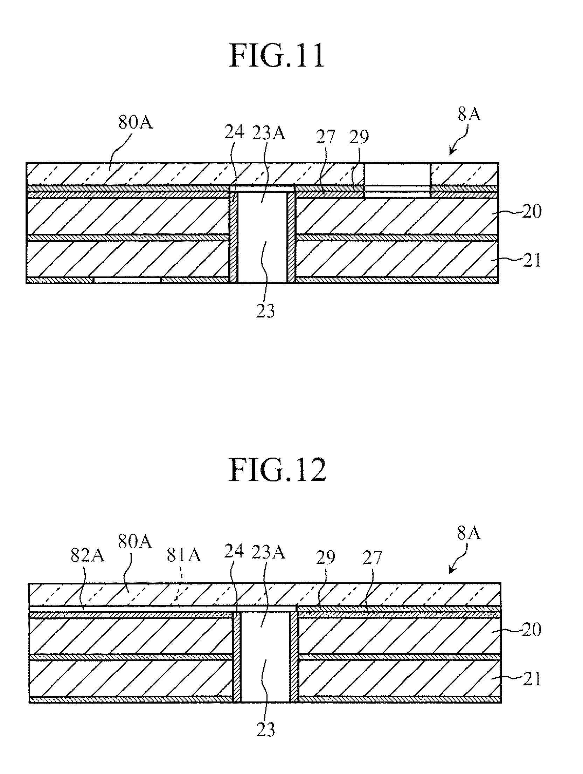

The biosensor can variously be modified as shown in Figs. 9 to 27. In the drawings referred to below, the same elements as the analysis apparatus 1 and the biosensors 2 explained previously with reference to Figs. 1 to 7 are designated with the same symbols, and redundant explanation will be omitted below.

A biosensor 8A shown in Figs. 9 to 12 includes a cover 80A and a discharge passage 81A.

The cover 80A seals the opening 23A of the capillary 23. The cover 80A entirely covers the insulation layer 27 which covers the working electrode 20. The cover 80A is made of material through which laser beam can pass, e.g., transparent glass and PET.

The discharge passage 81A is defined by a slit 82A of an insulation layer 29. The slit 82A is formed up to an edge of the insulation layer 29, and is connected to the capillary 23. That is, the discharge passage 81A can discharge gas in the capillary 23.

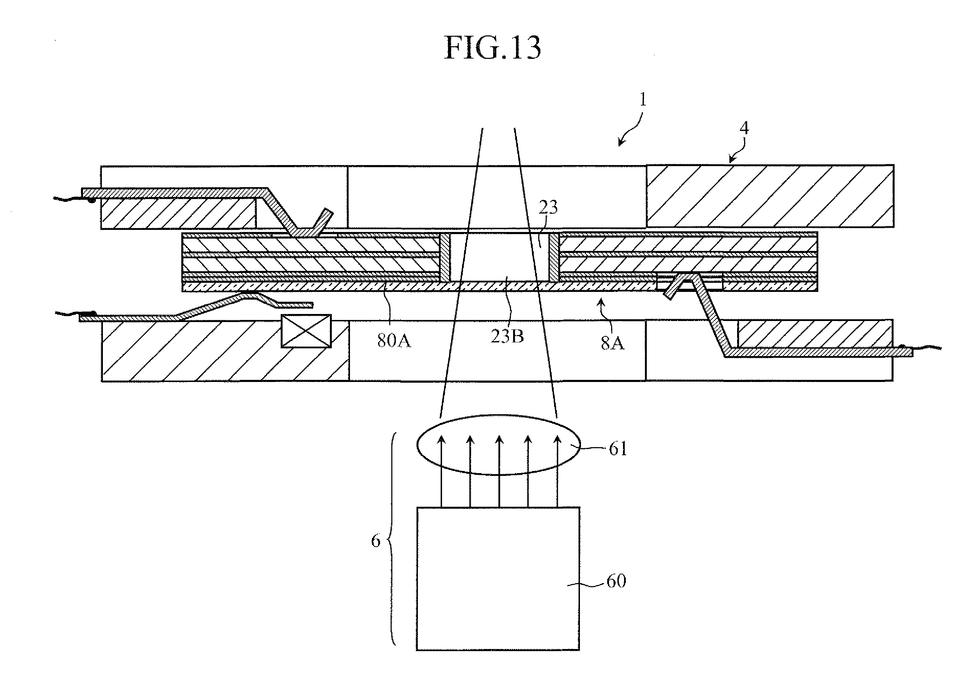

As shown in Fig. 13, the biosensor 8A is mounted on the connector 4 of the analysis apparatus 1 such that the opening 23B of the capillary 23 sealed by the cover 80A is located on the incident side of the laser beam. That is, the biosensor 8A can suppress the contamination on a light-emitting surface of the condensing lens 61 or the laser beam oscillator 60 in the lasing mechanism 6 that may be caused by fume generated when a skin is irradiated with laser beam or by scattering of blood or skin. Therefore, a skin can appropriately be irradiated with laser beam.

The single-use biosensor 8A prevents contamination caused by fumes, blood or skin from adhering and thus, it is unnecessary to clean the condensing lens 61. Thus, a load on a user is reduced, and nonuniform values of laser output generated when the condensing lens 61 is cleaned can be suppressed.

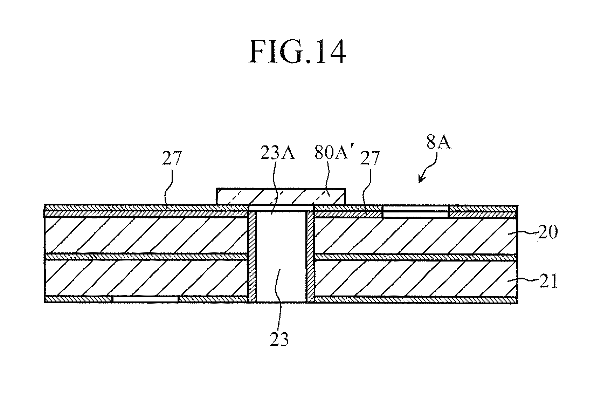

As shown in Fig. 14, it is unnecessary that the cover 80A' covers the entire working electrode 20 (insulation layer 27), and may selectively seal the opening 23A in the capillary 23.

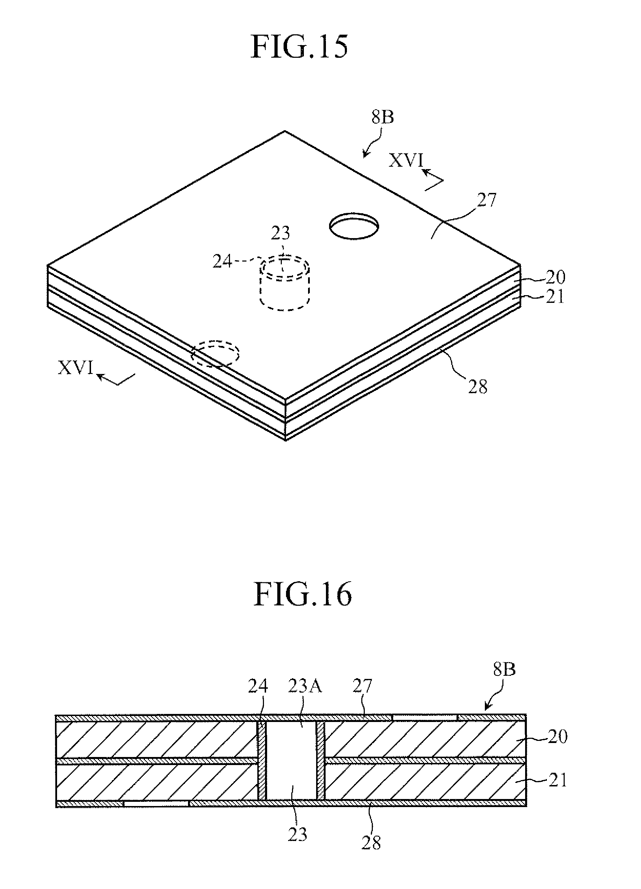

In a biosensor 8B shown in Figs. 15 and 16, through holes 27A and 28A (see Fig. 4) in the insulation layers 27 and 28 are omitted, and the openings 23A and 23B of the capillary 23 are sealed by the insulation layers 27 and 28.

By forming the opening in the insulation layers 27 and 28, the capillary 23 can exhibit the capillary action. The opening may be formed in the insulation layers 27 and 28 by laser beam to be emitted to the skin. In this case, the insulation layers 27 and 28 are made of material in which wavelength of laser beam is absorbed. For example, when laser beam having wavelength of 1500 to 3000 nm is employed, acrylic-based adhesive tape or polyester-based hot melt can be employed as material for forming the insulation layers 27 and 28. The openings may be formed in the insulation layers 27 and 28 by laser beam by including coloring agent or pigment that absorb wavelength of laser beam in the insulation layers 27 and 28.

In the biosensor 8B, the reagent layer 24 is sealed in the capillary 23 until bodily fluid issues from a skin. Thus, it is possible to prevent the reagent layer 24 from being deteriorated by moisture.

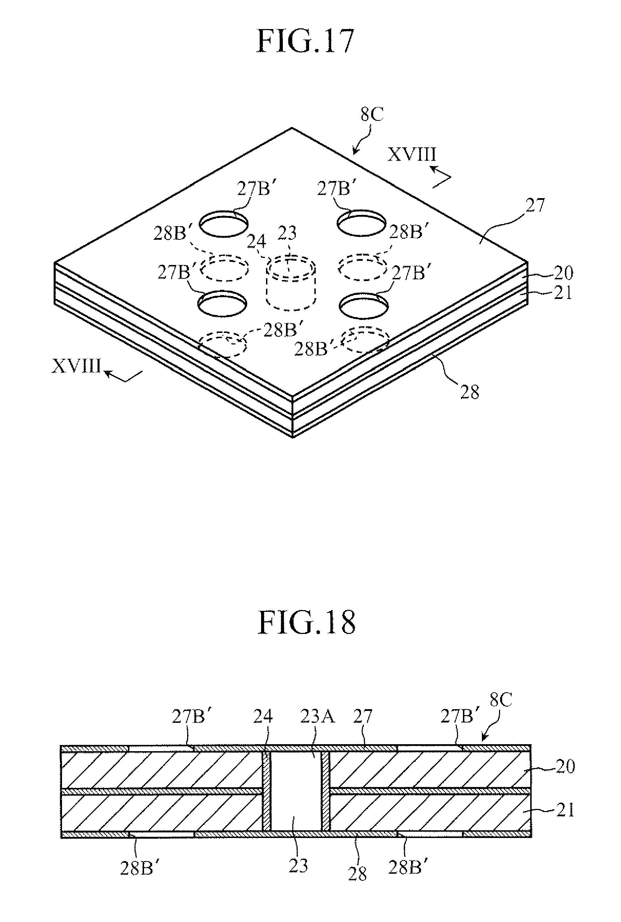

In a biosensor 8C shown in Figs. 17 and 18, the working electrode 20 and the counter electrode 21 have the same or almost the same thicknesses and shapes as viewed from above, and the insulation layers 27 and 28 have a plurality of holes 27B' and 28B' through which the working electrode 20 or the counter electrode 21 is exposed.

The plurality of holes 27B' and 28B' are disposed symmetrically with respect to a center line (line passing through a center) as viewed from above the biosensor 8C, the holes 27B' of the insulation layer 27 and the holes 28B' of the insulation layer 28 are formed at the same or almost the same positions as viewed from above.

Two surfaces, i.e., a front surface and a back surface of this biosensor 8C are the same, and any one of the two electrodes 20 and 21 can be used as the working electrode 20 or the counter electrode 21. Therefore, it is possible to prevent the biosensor 8C from being erroneously mounted on the analysis apparatus 1 (see Figs. 1 and 2). Since the front surface and the back surface of this biosensor 8C are the same, it is unnecessary to pay attention which surface is a front or back surface when a plurality of biosensors 8C are to be packed at the time of manufacturing, it is easy to align the biosensors 8C.

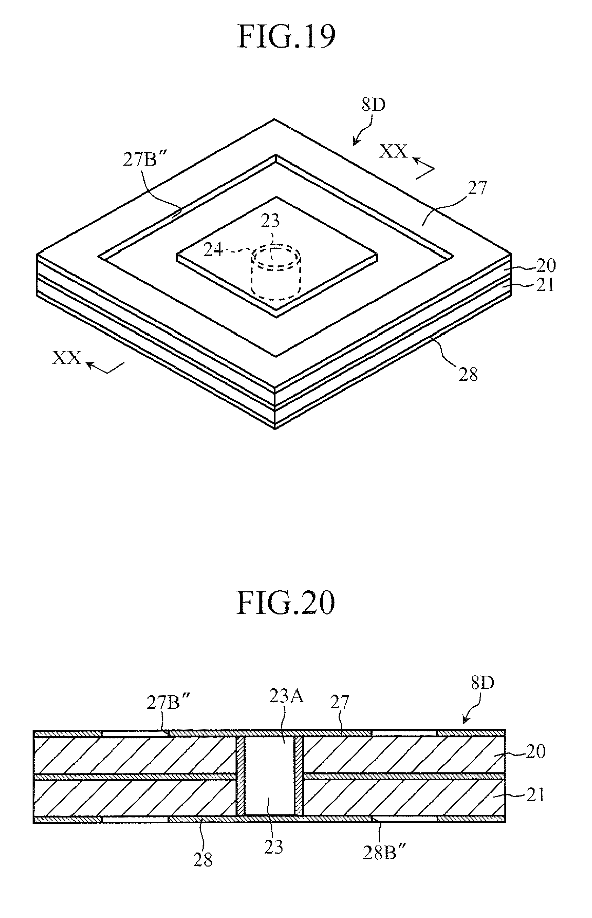

According to a biosensor 8D shown in Figs. 19 and 20, the working electrode 20 and the counter electrode 21 are exposed from holes 27B" and 28B" which surround the capillary 23.

According to this biosensor 8D also, since the working electrode 20 and the counter electrode 21 are exposed symmetrically with respect to the center line (line passing through a center) as viewed from above, a front surface and a back surface of the biosensor 8D are the same like the biosensor 8C (see Figs. 17 and 18).

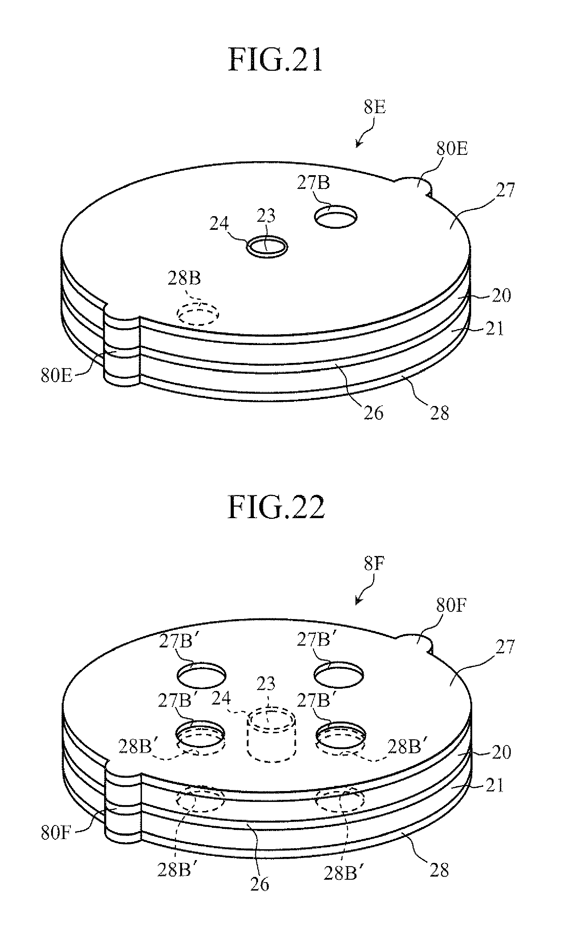

A biosensor 8E shown in Fig. 21 is formed a circular in shape as a whole, and includes positioning convex portions 80E.

By providing the biosensor 8E with the positioning convex portions 80E, it becomes easy to align a plurality of biosensors 8E when the biosensors 8E are packed at the time of manufacturing, and in the analysis apparatus 1 (see Figs. 1 and 2), it is possible to transfer and position the biosensor 8E to and with respect to the connector 4 (see Fig. 5).

According to a biosensor 8F shown in Fig. 22, like the biosensor 8C shown in Figs. 17 and 18, a plurality of holes 27B' and 28B' are disposed symmetrically with respect to a center line (line passing through the center) of the biosensor 8F as viewed from above, and the plurality of holes 27B' of the insulation layer 27 and the plurality of holes 28B' of the insulation layer 28 are disposed at the same or almost the same positions as viewed from above. This biosensor 8F is also provided with positioning convex portions 80F. Since a front surface and a back surface of the biosensor 8F are the same, this is convenient when the biosensor 8F is produced and used.

The biosensors 8E and 8F shown in Figs. 21 and 22 may have positioning concave portions instead of the positioning convex portions 80E and 80F.

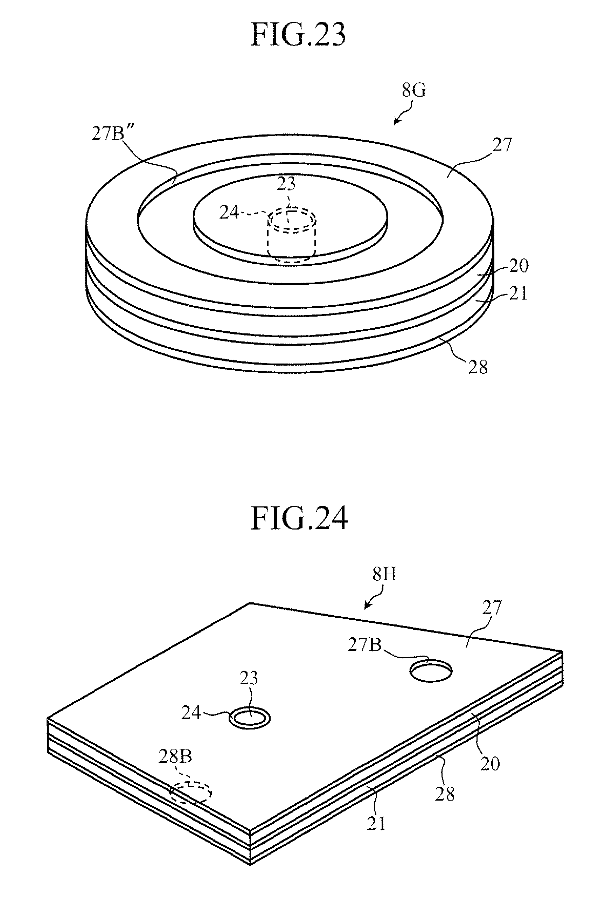

According to a biosensor 8G shown in Fig. 23, like the biosensors 8E shown in Figs. 19 and 20, the working electrode 20 and the counter electrode 21 are exposed from an annular hole 27B" formed in the insulation layers 27 and 28 such as to surround the capillary 23. Although only the insulation layer 27 has the hole 27B" in Fig. 23, a hole 28B" is formed also in the insulation layer 28. Since a front surface and a back surface of the biosensor 8GF are the same, this is convenient when the biosensor 8F is produced or used. Since the biosensor 8G can be mounted on the connector 4 (see Fig. 5) of the analysis apparatus 1 without taking the orientation of the biosensor 8G into account, the positioning convex portions 80E and 80F can be omitted as in the biosensors 8E and 8F.

A biosensor 8H shown in Fig. 24 is formed into a trapezoidal shape as viewed from above. Since the biosensor 8H is formed asymmetrical as viewed from above, when a plurality of biosensors 8H are to be packed at the time of manufacturing, the biosensors 8H can easily be aligned.

The shape of the biosensor which is asymmetric as viewed from above is not limited to the trapezoidal shape, and other shapes may be employed.

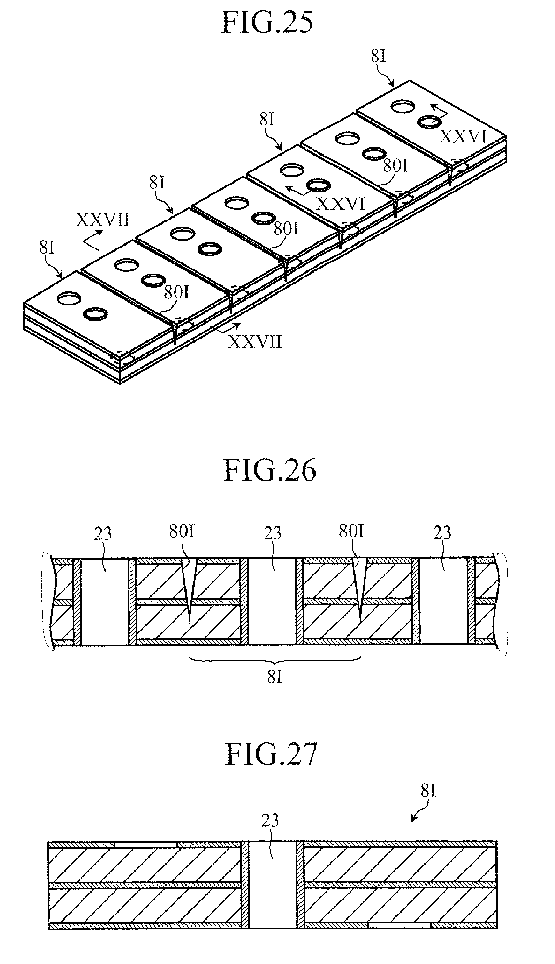

Figs. 25 to 27 show another example, and a plurality of biosensors 8I are connected in a form of an array, and cutting slits 80I are provided between adjacent biosensors 8I.

According to the array of the biosensors 8I, one of the biosensors 8I may be cut and mounted on the analysis apparatus and used, or the array may be set on the analysis apparatus as it is and may be used. In the case of the latter situation, the analysis apparatus is provided with a mechanism for moving the array. A user may cut a used biosensor 8I and discard, or the used biosensor SI may be automatically cut and discarded in the analysis apparatus.

## Claims

1. An analysis system comprising a laser beam oscillator which emits laser beam for issuing bodily fluid from a skin, one or more analysis tools which are used for analyzing specific ingredient in the bodily fluid and have a through hole through which the laser beam passes, wherein
the analysis tool includes a plurality of conductors laminated in a state where they are electrically insulated from each other.

2. The analysis system according to claim 1, wherein the analysis tool further includes a first insulation layer interposed between adjacent conductors, and one or a pair of second insulation layers which covers an outer surface of at least one of two outermost conductors located on the outermost portions among the plurality of conductors.

3. The analysis system according to claim 2, wherein the second insulation layer includes one or more holes from which a surface of the outermost conductor is exposed.

4. The analysis system according to claim 3, wherein the analysis tool has symmetric shapes with respect to a center line as viewed from above, and
the plurality of holes are formed at symmetric positions with respect to the center line.

5. The analysis system according to claim 4, wherein the two outermost conductors have the same or almost the same thicknesses, and
a plurality of holes in the pair of second insulation layers are formed at the same or almost the same positions as viewed from above.

6. The analysis system according to claim 1, wherein the analysis tool has one or more positioning portions.

7. The analysis system according to claim 3, wherein the through hole is formed at a center or substantially a center of the analysis tool, and
one of the holes is annularly formed such as to surround the through hole.

8. The analysis system according to claim 1, wherein the analysis tool is formed asymmetrically as viewed from above.

9. The analysis system according to claim 8, wherein the analysis tool is formed into a trapezoidal shape as viewed from above.

10. The analysis system according to claim 2, wherein the one or the pair of second insulation layers hermetically close an interior of the through hole.

11. The analysis system according to claim 10, wherein the one or the pair of second insulation layers are formed with openings by the laser beam.

12. The analysis system according to claim 1, wherein the analysis tool further includes a heating element for heating the plurality of conductors.

13. The analysis system according to claim 1, further comprising a connector having a plurality of terminals which are brought into contact with the plurality of conductors.

14. The analysis system according to claim 13, wherein the plurality of analysis tools are disposed in a state where they are laminated on one another, and the analysis tool further includes an analysis tool supply mechanism for supplying analysis tools to the connector one by one.

15. The analysis system according to claim 14, wherein at least one of the plurality of conductors is made of magnetic material, and the analysis tool supply mechanism electromagnetically supplies the analysis tool to the connector.

16. The analysis system according to claim 15, wherein the analysis tool supply mechanism includes first and second electromagnets, at least a portion of the analysis tool is magnetized by the first electromagnet and then, a polarity of the first electromagnet is reversed, repulsion is generated to the analysis tool, a polarity of the second electromagnet is set opposite from that of the first electromagnet, and an attraction force is generated between to the analysis tools.

17. The analysis system according to claim 1, wherein the plurality of analysis tools are connected to one another in a form of an array.

18. The analysis system according to claim 17, wherein the plurality of analysis tools are connected to one another in the form of the array in a state where cutting slits are provided each between adjacent analysis tools.
